# EUROPÄISCHE PATENTSCHRIFT

(11) **EP 0 669 109 B1**
(45) Veröffentlichungstag und Bekanntmachung des Hinweises auf die Patenterteilung: **26.05.1999**
(21) Anmeldenummer: 94810120.9
(22) Anmeldetag: 28.02.1994
(51) Int. Cl.: A61B 17/60, A61F 2/44

(54) **Stabilisierung von benachbarten Rückenwirbeln**
Stabilizer for adjacent vertebrae
Stabilisateur pour des vertèbres adjacentes

(43) Veröffentlichungstag der Anmeldung: 30.08.1995
(73) Patentinhaber: Sulzer Orthopädie AG, 6340 Baar (CH)
(72) Erfinder: Baumgartner, Walter, CH-9500 Wil (CH); Freudiger, Stefan, CH-3047 Bremgarten (CH); Dubois, Gilles, F-31240 St. Jean (FR)
(74) Vertreter: Heubeck, Bernhard

(56) Entgegenhaltungen:
- EP-A- 0 322 334
- EP-A- 0 348 272
- EP-A- 0 516 567
- WO-A-93/20771
- "Restauration cinématique de la précontrainte postérieure du Rachis Lombaire" Publikation von Dr. Jean-Philippe Lemaire

## Beschreibung

Die Erfindung handelt von einer Stabilisierung von benachbarten Rückenwirbeln umfassend ein Band und mindestens zwei Pedikelschrauben die jeweils in einem anderen Wirbel in Richtung ihrer Schraubenachse verankerbar sind, die einen Schraubenkopf mit einem Durchbruch quer zur Schraubenachse aufweisen, durch den das Band einziehbar ist, und die jeweils eine Klemmschraube aufweisen, um das Band quer zum Durchbruch in Richtung der Schraubenachse zu fixieren, sowie umfassend ein auf das Band aufgezogenes Stützelement.

Verstärkungen zwischen Rückenwirbeln werden in einer Publikation von Dr. Jean-Philippe Lemaire ("Restauration cinématique de la précontrainte posterieure du Rachis Lombaire"; Biomat, 17, rue Maryse Bastié - BP 12, F-91430 Igny) gezeigt. Es sind dort Systeme aus Bändern und Schrauben beschrieben, mit denen die bei der Bewegung der Wirbelsäule notwendige Spannung im posterioren Bereich der Lendenwirbel wieder hergestellt wird. Dabei werden im untersten Bereich der Lendenwirbel zum Kreuzbein hin weiche Unterlagen auf die Bänder aufgezogen, die beim Liegen oder Anlehnen des Patienten im Bereich des Kreuzbeins verhindern, dass sich die Schraubenköpfe der untersten Pedikelschrauben in das sie überdeckende Gewebe bohren.

Ebenso zeigt die Patentanmeldung WO 91/16018 eine Vorrichtung zum Erzeugen von Zugspannungen zwischen zwei Wirbeln im posterioren Bereich, indem Bänder zwischen Pedikelschrauben verspannt werden. Solche Vorrichtungen können im besten Fall eine ständige Zugspannung erzeugen.

In der Patentanmeldung EP-A-0 516 567 wird ein länglicher Dämpfungskörper gezeigt, der an jedem Ende einen Hals mit einem angeformten Kugelkopf besitzt. Dadurch, dass der Dämpfungskörper zwischen den Kugelköpfen in sich vorgespannt ist, wird dessen Steifigkeit erhöht. Im Kopf der Pedikelschrauben ist ein Gewindesackloch mit einem seitlich durchgehenden Schlitz angebracht, der so gross ist, dass der Hals mit Spiel eingebracht werden kann. Die eigentliche Kraftübertragung erfolgt zwischen einer konkaven Fläche im Grund des Gewindesacklochs und einer entgegengesetzt angeordneten konkaven Fläche in einer Halteschraube, wobei der Kugelkopf zwischen den konkaven Flächen passend gefangen sein soll, um eine Art Kugelgelenk zu bilden, welches die Bewegung zwischen den Wirbeln erleichtert. Der mit jeder Bewegung verbundene Verschleiss wird in Kauf genommen. Bei einer Verbindung von mehr als zwei benachbarten Wirbeln, müssen die Kugeln an mittleren Wirbeln halbiert sein, um sie zu einer zweiteiligen Kugel zusammenzusetzen und es müssen Sacklöcher mit zwei um etwa 180° versetzten Schlitzen vorgesehen werden. Dem Operateur, der die Pedikelschrauben nach der Beschaffenheit und Lage der Wirbel setzt, muss ein ganzes Arsenal von Dämpfungskörpern vorliegen, deren Kugelköpfe in enger Stufung unterschiedliche Abstände aufweisen, wobei für jeden Abstand Dämpfungskörper mit zwei vollen Kugeln, mit einer halben Kugel und einer vollen Kugel und mit zwei halben Kugeln vorhanden sein müssen.

In der Patentanmeldung EP-A-0 348 272 sind ebenfalls Pedikelschrauben mit einem Kopf gezeigt, der ein Gewindesackloch in der Richtung der Schraubenachse und quer dazu einen durchgehenden Schlitz mit einer Weite, kleiner als es der Gewindedurchmesser ist, aufweist, um in die Schlitze Verbindungsstangen einzulegen, die mit Schrauben befestigbar sind. Eine solche Lösung ist eine starre Festlegung der Wirbel. Sie bedingt sehr grosse Dimensionen vom Kopf der Pedikelschrauben, damit die durch den Schlitz geteilten Kopfhälften beim Anziehen der Befestigungsschrauben nicht unzulässig auseinandergespreizt werden.

Andere Vorrichtung benutzen, wie in der Offenlegungsschrift FR 2 615 095 beschrieben, Stangen die parallel zur Wirbelsäule verlaufen, um daran die einzelnen Wirbel mit Klemmvorrichtungen auszurichten, was zu einem starren Gebilde führt und den Patienten entsprechend unbeweglich macht.

Aufgabe der Erfindung ist es, eine einfache und bewegliche Stabilisierung zwischen Rückenwirbeln zu schaffen.

Diese Aufgabe wird mit den Kennzeichen vom Anspruch 1 gelöst, indem das Stützelement einen druckfesten Körper bildet zur Uebertragung von Druckkräften zwischen den beiden Schraubenköpfen, der Querschnitt des Bandes in passenden Bohrungen von Stützelement und Schraubenkopf allseitig anliegt, um Stützelement und Schraubenkopf zueinander zu zentrieren und indem das Band zwischen zwei benachbarten Pedikelschrauben über eine ausserhalb der Pedikelschrauben vorstehende Verlängerung vorspannbar ist, um Stützelement und Schraubenkopf auf einer ihnen gemeinsamen, um das Band herum verteilten Stützfläche gegenseitig abstützen zu können.

Die Erfindung hat den Vorteil, dass zwei benachbarte Wirbel aus einer vorgegebenen Ruhelage mit einer vorgegebenen Vorspannung gegen Zug posterior entlastet werden können, ohne dass eine dazwischenliegende Bandscheibe in der Ruhelage ständig unter Druck steht, während Druckkräfte posterior abgefangen und über Stützelemente übertragen werden. Dabei ist immer noch eine Restbeweglichkeit erhalten, weil Stützelemente und Schraubenköpfe im Zusammenwirken mit dem Band eine Art Gelenk bilden, welches mit zunehmender Auslenkung einen zunehmenden Widerstand gegen diese Auslenkung bildet. Es entsteht somit eine durch die Länge der Stützelemente vorgegebene Ruhestellung, aus der heraus gegen eine durch die Vorspannung des Bandes bestimmte Rückstellkraft ein beschränktes Vorbeugen und seitliches Verdrehen zwischen den Wirbeln stattfinden kann. Ebenso können die Schraubenachsen von zwei durch ein Stützelement verbundenen Pedikelschrauben leicht windschief zueinanderstehen.

Vorteilhafte Weiterbildungen der Erfindung sind in den abhängigen Ansprüchen 2 bis 14 gezeigt. Der gelenkartige Charakter der Verbindung zwischen Stützelement und Pedikelschraube wird durch eine gemeinsame, ringförmige um das Band ausgebildete Stützfläche noch verstärkt.

Bei einem metallischen oder festen nicht metallischen Werkstoff für das Stützelement lohnt es sich, die gemeinsame Stützfläche sphärisch auszubilden, um ein Schwenken in jeder Richtung bei genügend tragender Fläche zu ermöglichen. Dabei kann der Mittelpunkt der gemeinsamen Stützfläche entweder auf der Seite des Stützelements oder auf der Seite vom Schraubenkopf der Pedikelschraube liegen. Da sich der Abstand zwischen zwei in Wirbel eingeschraubten Pedikelschrauben nicht zum voraus genau bestimmen lässt, müssen die Stützelemente wie bei einem Baukasten in verschiedenen Längen zur Auswahl vorliegen.

Bei einem elastischen Stützkörper aus Kunststoff z.B. aus Polyurethan genügt es, wenn am Schraubenkopf eine ebene Ringfläche oder eine nach innen konische Ringfläche als Stützfläche angebracht ist, da der Kunststoff nachgeben kann und im wesentlichen nur auf Druck beansprucht wird. Solange die Stützfläche nur einem flachen Konusring oder einem flachen sphärischen Ring entspricht, wird der Stützkörper darin zentriert und passt sich wegen seiner Elastizität unter Vorspannung der Stützfläche an, auch wenn er als zylindrischer Hohlkörper ausgebildet ist. Ein weiterer Vorteil eines elastischen Stützkörpers besteht darin, dass er aus der Ruhestellung heraus, in welcher die Zugkräfte kompensiert werden, druckabhängig elastisch nachgibt, was zu einer Dämpfung bei stossartigen Belastungen führt.

Für solche elastische zylindrische Hohlkörper lässt sich die Vielfalt eines Baukastens einfach erreichen, indem ein zylindrischer Hohlkörper durch radiale Einschnitte von aussen in zylindrische Teilstücke unterteilt ist, welche durch einen Hals miteinander verbunden sind. Der radiale Einschnitt und der Hals sind so bemessen, dass Teilstücke während der Operation und vor dem Einführen des Bandes mit einem Skalpell abgetrennt werden können, um die richtige Länge zu erreichen. Um dem Stützelement eine bessere Knickfestigkeit zu verleihen ist es vorteilhaft, wenn es mittig eine bauchige Form aufweist. Die selbstschneidenden Pedikelschrauben haben ein Gewinde mit konstantem Aussendurchmesser und mit einem konisch wachsenden Kerndurchmesser. Der Schraubenkopf besteht aus einem Kugelausschnitt mit Mittelpunkt auf der Schraubenachse, um wenig Raum zu beanspruchen.

In den Figuren sind verschiedene Ausführungsbeispiele der Erfindung aufgeführt. Es zeigen:
- Fig. 1: schematisch einen Querschnitt durch einen Lendenwirbel mit einer eingeschraubten Pedikelschraube;
- Fig. 2: schematisch die Seitenansicht von zwei Pedikelschrauben, die über ein Stützelement und ein Band miteinander verbunden sind;
- Fig. 3: schematisch die Draufsicht auf einen der seitlich abgeflachten Schraubenköpfe aus Fig. 2;
- Fig. 4: schematisch die Seitenansicht von zwei Pedikelschrauben, die über ein Stützelement und ein Band miteinander verbunden sind;
- Fig. 5: schematisch die Draufsicht auf einen der runden Schraubenköpfe aus Fig. 4;
- Fig. 6, 7: schematisch im nicht vorgespannten Zustand die Seitenansicht von zwei Pedikelschrauben, die über ein elastisches Stützelement und ein Band miteinander verbunden sind;
- Fig. 8: schematisch im Schnitt ein elastisches Stützelement nach Fig. 6, das zwischen zwei Pedikelschrauben verpresst ist; und
- Fig. 9: schematisch ein Stützelement aus Fig. 8 im unverpressten Zustand.

In den Figuren sind Stabilisierungen zwischen zwei in benachbarten Wirbeln befestigten Pedikelschrauben 2, 3 gezeigt. Zwischen den Schraubenköpfen 6 wird ein auf ein Band 1 aufgezogenes Stützelement 10, welches wahlweise aus einem elastischen oder starren Werkstoff besteht, fixiert, indem das Band an den Schraubenköpfen 6 unter Vorspannung befestigt wird. Das Band 1 weist einen gegen Scherkräfte widerstandsfähigen runden Querschnitt auf und besteht aus elastischem Kunststoff, während das Stützelement 10 einen druckfesten Körper zur Uebertragung von Druckkräften zwischen den beiden Schraubenköpfen 6 bildet. Stützelement 10 und Schraubenkopf 6 liegen mit passenden Bohrungen 9, 12 allseitig am Band 1 an, um sich gegenseitig zu zentrieren und stützen sich gegenseitig auf einer ihnen gemeinsamen um das Band 1 herum verteilten Stützfläche 13 ab.

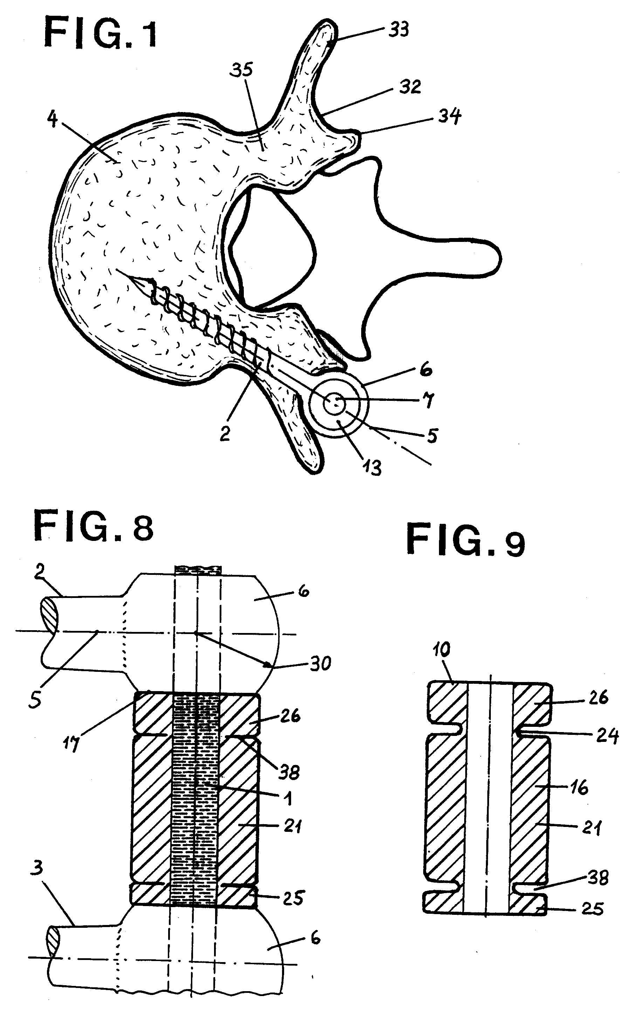

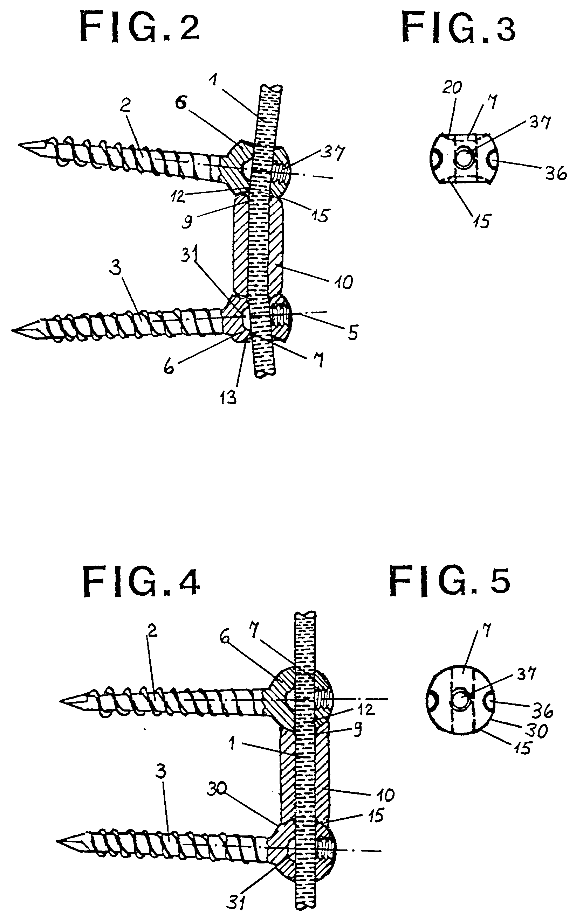

In Figur 1 ist eine Pedikelschraube 2 im Sattel 32 zwischen dem Processus costalis 33 und dem Processus mamillaris 34 so eingeschraubt, dass sie mit ihrer Achse 5 durch das Pedikel 35 den Wirbelkörper 4 zur Verankerung erreicht. Der Schraubenkopf 6 ist Ausschnitt aus einem Kugelkörper und liegt im Sattel 32 auf. Der Schraubenkopf 6 ist an zwei gegenüberliegenden Stützflächen 13 abgeflacht und weist einen Durchbruch 7 auf, durch den ein Band einziehbar ist. Die Pedikelschraube 2, 3 in benachbarten Wirbelkörpern sind so ausgerichtet, dass sich die Stützflächen 13 gegenüberliegen, um dazwischen einen auf das Band 1 aufziehbaren Stützkörper wie in den Figuren 2, 4, 6, 7, 8 einzubringen.

Im Beispiel von Fig. 2, 3 ist im Schraubenkopf 6 der Pedikelschrauben 2, 3 eine Durchgangsbohrung 12 angebracht, die in einer Bohrung 9 des Stützelements 10 fortgesetzt ist, um das Band 1 aufzunehmen, welches unter Vorspannung über Schrauben in Gewindebohrungen 37 in gegenüberliegenden Vertiefungen 31 geklemmt wird. Die Stützflächen 13 bestehen am Schraubenkopf aus konkaven Ringflächen 20, die im Fall eines starren Stützkörpers 10 sphärisch 15 mit einem Radius grösser 5 mm sind, während der Stützkörper eine passende sphärische Gegenfläche 15 aufweist. Am Schraubenkopf sind Aussparungen 36 angebracht, um ein Eindrehwerkzeug oder ein Halteinstrument ansetzen zu können.

Im Beispiel von Fig. 4, 5 ist der Schraubenkopf mit seiner Kugelfläche 30 gleichzeitig Stützfläche für eine entsprechende konkave Gegenfläche am Stützkörper 10. Die restlichen Elemente entsprechen denen von Fig. 2, 3.

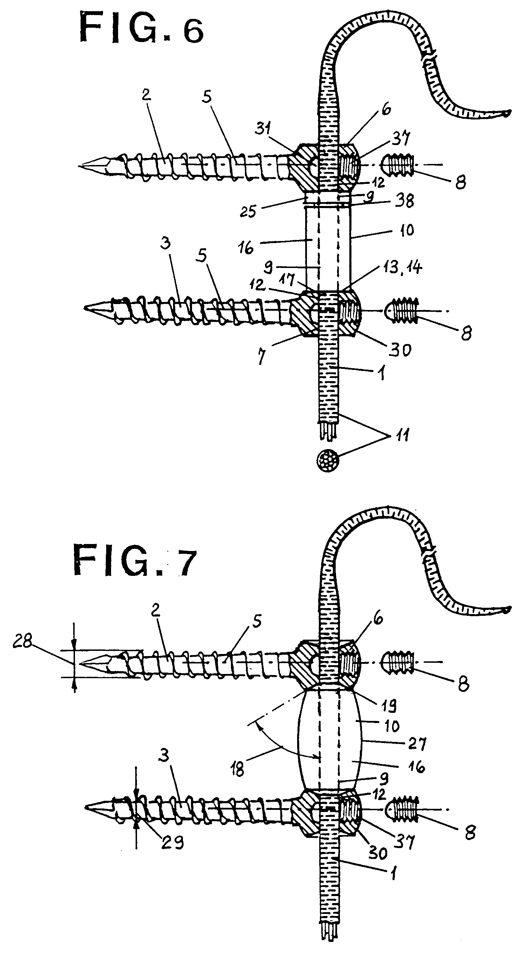

Im Beispiel von Fig. 6, 8, 9 besteht das Stützelement aus einem zylindrischen Hohlkörper 21 aus elastischem Kunststoff 16, welcher im unbelasteten Zustand durch jeweils einen umlaufenden radialen Einschnitt 38 von aussen in zwei zusätzliche Teilstücke 25, 26 unterteilt ist. Die Teilstücke sind durch einen Hals 24 miteinander verbunden, wobei der Hals so schwach ist, dass er sich unter axialer Vorspannung vollständig komprimiert, damit die Flächen vom Einschnitt 38 zum Tragen kommen. Der Einschnitt 38 ist nur so breit, dass im unbelasteten Zustand Teilstücke 25, 26 mit einem Skalpell abtrennbar sind, um das Stützelement 10 auf die richtige Länge zu kürzen. Dabei hat sich eine Ausführung mit einem Mittelteil und zwei Teilstücken 25, 26 als zweckmässig erwiesen, um vier mögliche Einbaulängen zu erhalten, die gleichmässig gestuft sind, wenn ein Teilstück 25 die halbe Länge vom anderen Teilstück 26 aufweist. Es können daraus vier Längen gewonnen werden, nämlich "mittleres Teilstück und Teilstücke 25, 26"; "mittleres Teilstück und Teilstück 26"; "mittleres Teilstück und Teilstück 25" und "mittleres Teilstück", die eine gleiche Stufung aufweisen. Die Länge vom Hals 24 darf dabei nicht mitgerechnet werden. In Fig. 6 ist ein Stützelement mit einem Teilstück 25 gezeigt. Das Band 1 ist eingezogen, aber noch unverspannt. Als nächster Schritt würde z.B. ein Gewindestift 8 an der Pedikelschraube 3 angezogen, um das Band 1 zu fixieren. Anschliessend wird zwischen der oberen Pedikelschraube 2 und dem Band 1 eine passende Vorspannung angebracht und das vorgespannte Band mit dem oberen Gewindestift 8 fixiert. Die Stützflächen 13 sind als ebene ringförmige Flächen 14, 17 ausgeführt. Um das Stützelement besser zu zentrieren, kann diese Ringfläche am Schraubenkopf als konische Ringfläche 19 ausgeführt sein, solange sich das Stützelement 10 im Rahmen seiner Elastizität hineinverformt.

Im Beispiel von Fig. 7 sind die Stützflächen am Schraubenkopf 6 und am Stützelement 10 konisch, wobei der halbe Konuswinkel 18 mehr als 45° beträgt. Gleichzeitig weist der Stützkörper zur Mitte hin eine bauchige Form 27 auf, um seine Knickfestigkeit zu erhöhen. Auch in diesem Fall sind Teilstücke 25, 26 - wie vorher besprochen - möglich, wenn die radialen Einschnitte auf Kegelmantelflächen mit ähnlichem Konuswinkel wie Konuswinkel 18 liegen.

Die in den Figuren 3 und 5 dargestellten Aussparungen 36 für ein Einschraubwerkzeug dienen nicht nur zum Einziehen der Pedikelschrauben, welche selbstschneidend mit einem Gewinde mit konstantem Aussendurchmesser 28 und mit einem wachsenden Kerndurchmesser 29 ausgeführt sind, sondern sind auch Ansatzflächen zum Erzeugen eines Gegenmomentes beim Anziehen der Gewindestifte 8.

Bei den aufgeführten Beispielen weist das Band 1 einen gegen Scherkräfte widerstandsfähigen runden Querschnitt 11 auf, wie ihn zum Beispiel umflochtene künstliche Kreuzbänder besitzen können. Bei einer Biegebelastung zwischen Schraubenkopf 6 und Stützkörper 10 wird daher ein Rückstellmoment erzeugt, indem das Band 10 sich dehnt, während sich der Druck in der Stützfläche 13 einseitig nach aussen verlagert. Dies hat den Vorteil, dass die Verstärkung aus einer möglichen elastischen Deformation, die auch Biegung beinhaltet, immer eine Rückstellung in die gleiche Ruhelage anstrebt.

Der als Kugelausschnitt 30 ausgeführte Schraubenkopf beansprucht nur ein geringes Volumen, was ein tiefes Einbringen erleichtert, und bietet keine scharfen Kanten zum Nachbargewebe. Die Gewindestifte 8 sollten nach dem Einschrauben bündig mit der Kugelfläche 30 abschliessen.

Die hier beschriebene Stabilisierung ist nicht auf zwei benachbarte Wirbelkörper mit dazwischenliegender Bandscheibe begrenzt, sondern kann über mehrere aneinander anschliessende Wirbelkörper angebracht werden, um mehrere Segmente zu stabilisieren.

Ebenso können die Stützelemente 10 als leicht gekrümmte Rohre ausgeführt sein, um der individuellen Lage der einzelnen Pedikelschrauben besser gerecht zu werden. Die Stützflächen 13 von zwei Pedikelschrauben 2, 3 zu einem Stützelement 10 müssen sich dann weniger genau gegenüberliegen.

## Patentansprüche

1. Stabilisierung von benachbarten Rückenwirbeln umfassend ein Band (1), welches aus elastischem Kunststoff besteht und einen gegen Scherkräfte widerstandsfähigen runden Querschnitt (11) aufweist, und mindestens zwei Pedikelschrauben (2, 3) die jeweils in einem anderen Wirbel in Richtung ihrer Schraubenachse (5) verankerbar sind, die einen Schraubenkopf (6) mit einem Durchbruch (7) quer zur Schraubenachse (5) aufweisen, durch den das Band einziehbar ist, und die jeweils eine Klemmschraube (8) aufweisen, um das Band (1) quer zum Durchbruch (7) in Richtung der Schraubenachse (5) zu fixieren, sowie umfassend ein auf das Band (1) aufgezogenes Stützelement (10), dadurch gekennzeichnet, dass das Stützelement (10) einen druckfesten Körper zur Uebertragung von Druckkräften zwischen zwei Schraubenköpfen bildet, der Querschnitt des Bandes in passenden Bohrungen (9, 12) von Stützelement und Schraubenkopf allseitig anliegt, um Stützelement (10) und Schraubenkopf (6) zueinander zu zentrieren, und dass das Band (1) zwischen zwei benachbarten Pedikelschrauben über eine ausserhalb der Pedikelschrauben vorstehende Verlängerung vorspannbar ist, um Stützelement (10) und Schraubenkopf (6) auf einer ihnen gemeinsamen, um das Band (1) herum verteilten Stützfläche (13) gegenseitig abstützen zu können.

2. Stabilisierung nach Anspruch 1, dadurch gekennzeichnet, dass die gemeinsame Stützfläche (13) aus einer ringförmigen Fläche (14) um das Band (1) besteht.

3. Stabilisierung nach Anspruch 1 oder 2, dadurch gekennzeichnet, dass das Stützelement (10) aus Metall oder aus einem festen nicht-metallischen Werkstoff besteht.

4. Stabilisierung nach Anspruch 3, dadurch gekennzeichnet, dass die gemeinsame Stützfläche (13) ein Ausschnitt aus einer sphärischen Fläche (15) ist, die ihren Mittelpunkt auf der Seite vom Schraubenkopf (6) oder auf der Seite vom Stützelement (10) aufweist.

5. Stabilisierung nach Anspruch 3 oder 4, dadurch gekennzeichnet, dass ein Baukastensystem gebildet ist, indem mehrere Stützelemente (10) mit unterschiedlicher Länge auswählbar oder einstellbar sind.

6. Stabilisierung nach Anspruch 1 oder 2, dadurch gekennzeichnet, dass das Stützelement (10) aus einem elastischen Kunststoff (16) vorzugsweise aus Polyurethan besteht.

7. Stabilisierung nach Anspruch 6, dadurch gekennzeichnet, dass die Stützfläche (13) am Schraubenkopf (6) durch eine ebene Ringfläche (17) oder durch eine nach innen konische Ringfläche (19) mit einem halben Konuswinkel (18) grösser 45° oder durch eine konkave Ringfläche (20) mit einem sphärischen Radius grösser 5 mm gebildet ist.

8. Stabilisierung nach Anspruch 6 oder 7, dadurch gekennzeichnet, dass das Stützelement (13) aus einem zylindrischen Hohlkörper (21) besteht.

9. Stabilisierung nach Anspruch 8, dadurch gekennzeichnet, dass der zylindrische Hohlkörper (21) durch jeweils einen umlaufenden radialen Einschnitt (38) von aussen in zylindrische Teilstücke (25, 26) unterteilt ist, die durch einen Hals (24) miteinander verbunden sind.

10. Stabilisierung nach Anspruch 9, dadurch gekennzeichnet, dass der zylindrische Hohlkörper (21) aus einem mittleren und zwei äusseren Teilstücken (25, 26) besteht, wobei die äusseren Teilstücke (25, 26) mit einem im Einschnitt (38) geführten Skalpell abtrennbar sind und das eine äussere Teilstück (25) die halbe Länge des anderen äusseren Teilstücks (26) aufweist, um durch Abtrennen von keinem, von dem einen, von dem anderen oder von dem einen und dem anderen Teilstück, vier gleichmässig gestufte Einbaulängen zu erhalten.

11. Stabilisierung nach Anspruch 6 oder 7, dadurch gekennzeichnet, dass das Stützelement (10) eine mittig bauchige Form (27) aufweist, um die Knickfestigkeit zu erhöhen.

12. Stabilisierung nach einem der Ansprüche 1 bis 11, dadurch gekennzeichnet, dass die Pedikelschrauben (2, 3) selbstschneidend mit einem Gewinde mit konstantem Aussendurchmesser (28) und mit einem konisch wachsenden Kerndurchmesser (29) ausgeführt sind.

13. Stabilisierung nach einem der Ansprüche 1 bis 12, dadurch gekennzeichnet, dass der Schraubenkopf (6) aus einem Kugelausschnitt (30) besteht, um möglichst geringe Angriffsflächen zu geben.

14. Stabilisierung nach einem der Ansprüche 1 bis 13, dadurch gekennzeichnet, dass der Schraubenkopf (6) am Durchbruch (7) in Richtung der Schraubenachse (5) und entgegengesetzt zur Klemmschraube (8) eine Vertiefung (31) aufweist, in welche das Band (1) mit der Klemmschraube einpressbar ist.

15. Stabilisierung nach einem der Ansprüche 1 bis 14, dadurch gekennzeichnet, dass das Band (1), die Pedikelschrauben (2, 3) und Stützelemente (10) über mehrere Wirbel fortgesetzt sind, um einen ganzen Bereich zu verstärken.

## Claims

1. a stabiliser for adjacent thoracic vertebrae comprising a strap, which is made of elastic synthetic material and has a round cross-section (11) which is resistant to shearing forces, and at least two pedicular screws (2, 3) each of which may be anchored in a different vertebra in the direction of its screw axis (5) and each of which has a screw head (6) with a hole (7) transverse to the screw axis (5), through which may be threaded the strap, and each of which includes a clamping screw (8) to fix the strap (1) transversely to the hole (7) in the direction of the screw axis (5), and also including a support element (10) threaded onto the strap (1),
**characterised in that** the support element (10) forms a pressure-resistant body for the transmission of compressive forces between two screw heads, the cross-section of the strap bears on all sides in fitting holes (9, 12) in the support element and screw head in order to mutually centre the support element (10) and the screw head (6),
**and in that** the strap (1) can be prestressed between two adjacent pedicular screws over an extension continuing outside the pedicular screws in order to be able to support the support element (10) and the screw head (6) on a support area (13) which is common to them and is distributed around the strap (1).

2. a stabilizer according to Claim 1,
**characterised in that** the common support area (13) consists of an annular surface (14) around the strap (1).

3. A stabilizer according to Claim 1 or 2,
**characterised in that** the support element (10) is made of metal of a strong non-metallic material.

4. A stabilizer according to Claim 3,
**characterised in that** the common support area (13) is a portion of a spherical surface (15) which has its centre on the side of the screw head (6) or on the side of the support element (10).

5. A stabilizer according to Claim 3 or 4,
**characterised in that** a building block system is provided in that several support elements (10) of different lengths may be selected or adjusted.

6. A stabilizer according to Claim 1 or 2,
**characterised in that** the support element (10) is made from an elastic synthetic material, preferably of polyurethane.

7. A stabilizer according to Claim 6,
**characterised in that** the support area (13) on the screw head (6) is formed by a planar annular surface (17) or by an inwardly conical annular surface (19) with half of the cone angle (18) greater than 45° or by a concave annular surface (20) having a spherical radius greater than 5 mm.

8. A stabilizer according to Claim 6 or 7,
**characterised in that** the support element (13) consists of a cylindrical hollow body (21).

9. A stabilizer according to Claim 8,
**characterised in that** the cylindrical hollow body (21) is divided by peripheral radial incisions (38) from the outside into cylindrical portions (25, 26) interconnected by a neck (24).

10. A stabilizer according to Claim 9,
**characterised in that** the cylindrical hollow body (21) is composed of a central part and two outer portions (25, 26), wherein the outer portions (25, 26) are severable by a scalpel guided in the incision (38) and the one outer portion (25) is half the length of the other outer portion (26), so that by severing none, the one, the other or both portions, four uniformly stepped installation lengths are obtained.

11. A stabilizer according to Claim 6 or 7,
**characterised in that** the support element (10) is barrel-shaped (27) in the middle so as to increase its buckling strength.

12. A stabilizer according to one of Claims 1 to 11,
**characterised in that** the pedicular screws (2, 3) are tapping screws and have a thread with a constant external diameter (28) and a conically increasing core diameter (29).

13. A stabilizer according to one of Claims 1 to 12,
**characterised in that** the screw head (6) consists of a spherical portion (30) to provide application areas which are as small as possible.

14. A stabilizer according to one of Claims 1 to 13,
**characterised in that** the screw head (6) has at the hole (7) in the direction of the screw axis (5) and opposite the clamping screw (8) a depression (31) into which may be pressed the strap (1) by the clamping screw.

15. A stabiliser according to one of Claims 1 to 14,
**characterised in that** the strap (1), the pedicular screws (2, 3) and the support elements (10) extend over several vertebrae to strengthen an entire section.

## Revendications

1. Stabilisation de vertèbres dorsales avoisinantes comportant une bande (1) qui est réalisée en matière synthétique élastique et qui présente une section transversale ronde (11) résistant à des forces de cisaillement, et au moins deux vis de pédicule (2, 3) qui peuvent être ancrées respectivement dans une autre vertèbre dans la direction de leur axe de vissage (5), qui présentent une tête de vis (6) avec un perçage (7) transversalement à l'axe de vis (5), à travers lequel la bande peut être insérée, et qui présentent respectivement une vis de serrage (8) pour fixer la bande (1) transversalement au perçage (7) en direction de l'axe de vis (5) et comprenant un élément d'appui (10) monté sur la bande (1), caractérisée en ce que l'élément d'appui (10) forme un corps résistant à la pression pour transférer des forces de pression entre deux têtes de vis, que la section transversale de la bande s'applique de tout côté dans des perçages adaptés (9, 12) de l'élément d'appui et de la tête de vis pour centrer l'élément d'appui (10) et la tête de vis (6) l'un relativement à l'autre, et en ce que la bande (1) peut être précontrainte entre deux vis de pédicule avoisinantes par un prolongement dépassant à l'extérieur des vis de pédicule pour pouvoir supporter mutuellement l'élément d'appui (10) et la tête de vis (6) sur une surface d'appui (13) qui leur est commune, répartie autour de la bande (1).

2. Stabilisation selon la revendication 1, caractérisée en ce que la surface d'appui commune (13) est constituée d'une face annulaire (14) autour de la bande (1).

3. Stabilisation selon la revendication 1 ou 2, caractérisée en ce que l'élément d'appui (10) est réalisé en métal ou en un matériau solide non métallique.

4. Stabilisation selon la revendication 3, caractérisée en ce que la surface d'appui commune (13) est une découpure d'une surface sphérique (15) qui a son centre sur le côté de la tête de vis (6) ou sur le côté de l'élément d'appui (10).

5. Stabilisation selon la revendication 3 ou 4, caractérisée en ce qu'il est formé un système de boîte de construction en ce que plusieurs éléments d'appui (10) peuvent être sélectionnés ou sont ajustables à une longueur différente.

6. Stabilisation selon la revendication 1 ou 2, caractérisée en ce que l'élément d'appui (10) est constitué d'une matière synthétique élastique (16), de préférence en polyuréthane.

7. Stabilisation selon la revendication 6, caractérisée en ce que la surface d'appui (13) à la tête de vis (6) est formée par une surface annulaire plane (17) ou par une surface annulaire intérieure conique (19) avec un demi angle de cône (18) supérieur à 45° ou par une surface annulaire concave (20) avec un rayon sphérique supérieur à 5 mm.

8. Stabilisation selon la revendication 6 ou 7, caractérisée en ce que l'élément d'appui (13) est constitué d'un corps creux cylindrique (21).

9. Stabilisation selon la revendication 8, caractérisée en ce que le corps creux cylindrique (21) est divisé respectivement par une entaille radiale (38) s'étendant tout autour de l'extérieur en des pièces partielles cylindriques (25, 26) qui sont reliées les unes aux autres par un col (24).

10. Stabilisation selon la revendication 9, caractérisée en ce que le corps creux cylindrique (21) est constitué d'une pièce partielle médiane et de deux pièces partielles extérieures (25, 26), où les pièces partielles extérieures (25, 26) peuvent être séparées avec un scalpel guidé dans l'entaille (38), et en ce qu'une pièce partielle extérieure (25) a la moitié de la longueur de l'autre pièce partielle extérieure (26) pour obtenir par une séparation d'aucune, d'une, de l'autre ou d'une et de l'autre pièce partielle, quatre longueurs de montage uniformément échelonnées.

11. Stabilisation selon la revendication 6 ou 7, caractérisée en ce que l'élément d'appui (10) a une forme ventrue (27) au centre pour augmenter la résistance au flambage.

12. Stabilisation selon l'une des revendications 1 à 11, caractérisée en ce que les vis de pédicule (2, 3) sont réalisées d'une manière autotaraudeuse avec un filetage d'un diamètre extérieur constant (28) et d'un diamètre de noyau (29) croissant d'une manière conique.

13. Stabilisation selon l'une des revendications 1 à 12, caractérisée en ce que la tête de vis (6) est constituée d'un segment sphérique (30) pour présenter des surfaces d'attaque les plus réduites possibles.

14. Stabilisation selon l'une des revendications 1 à 13, caractérisée en ce que la tête de vis (6) présente aux perçages (7) en direction de l'axe de vis (5) et d'une manière opposée à la vis de serrage (8) un creux (31) dans lequel la bande (1) peut être enfoncée avec la vis de serrage.

15. Stabilisation selon l'une des revendications 1 à 14, caractérisée en ce que la bande (1), les vis de pédicule (2, 3) et les éléments d'appui (10) s'étendent sur plusieurs vertèbres pour renforcer tout une zone.
